(19) Europäisches Patentamt
European Patent Office
Office européen des brevets

(11) Veröffentlichungsnummer: **0 131 899**
**B1**

(12) # EUROPÄISCHE PATENTSCHRIFT

(45) Veröffentlichungstag der Patentschrift: **25.11.87**

(51) Int. Cl.[4]: **C 07 J 53/00**, A 61 K 31/585

(21) Anmeldenummer: **84108122.7**

(22) Anmeldetag: **11.07.84**

(54) **15,16-Methylen-17-alpha-pregna-4,6-dien-21-carbonsäuresalze, diese enthaltende pharmazeutische Präparate sowie Verfahren zu deren Herstellung.**

(30) Priorität: **15.07.83 DE 3326013**

(43) Veröffentlichungstag der Anmeldung: **23.01.85 Patentblatt 85/4**

(45) Bekanntmachung des Hinweises auf die Patenterteilung: **25.11.87 Patentblatt 87/48**

(84) Benannte Vertragsstaaten: **AT BE CH DE FR GB IT LI LU NL SE**

(56) Entgegenhaltungen:
**EP-A-0 019 690**
**EP-A-0 099 854**
**DE-A-2 264 189**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Nickisch, Klaus, Dr., Alt- Lichtenrade 11, D-1000 Berlin 49 (DE)**
Erfinder: **Bittler, Dieter, Bölkauer Pfad 11, D-1000 Berlin 27 (DE)**
Erfinder: **Laurent, Henry, Dr., Glambecker Weg 21, D-1000 Berlin 28 (DE)**
Erfinder: **Wiechert, Rudolf, Prof. Dr., Petzower Strasse 8A, D-1000 Berlin 39 (DE)**
Erfinder: **Losert, Wolfgang, Prof. Dr., Landshuter Strasse 22, D-1000 Berlin 30 (DE)**

**EP 0 131 899 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft 15,16-Methylen-17α-pregna-4,6-dien-21-carbonsäuresalze der allgemeinen Formel I

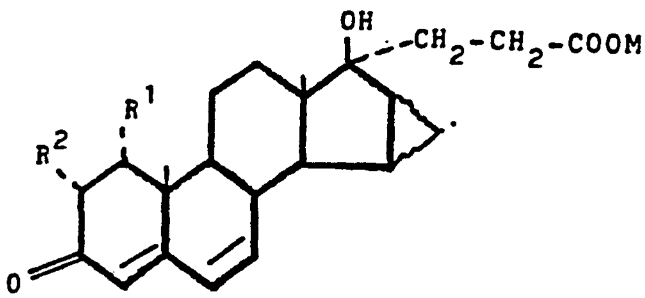

(I),

worin

$R^1$ und $R^2$ jedes für sich Wasserstoff oder gemeinsam eine weitere CC-Bindung oder eine Methylengruppe, und

M ein Alkalimetall

bedeuten

und die Methylengruppe in 15, 16-Stellung in α- oder β-Stellung steht; ferner diese enthaltende pharmazeutische Präparate sowie Verfahren zu deren Herstellung.

Die neuen Verbindungen der allgemeinen Formel I sind Alkalisalze der 3-substituierten Propionsäure. Als Alkalisalze kommen Kalium-, Natrium- oder Lithiumsalze, vorzugsweise Kaliumsalze, infrage.

Die neuen Verbindungen der allgemeinen Formel I haben die Eigenschaft, die Wirkung von Aldosteron oder Desoxycorticosteron auf die Natrium- und Kaliumsalzausscheidung aufzuheben bzw. umzukehren. Die neuen Verbindungen sind leicht wasserlöslich wie das bekannte Kaliumcanrenoat, sie sind dem Kaliumcanrenoat aber in der Antialdosteronwirkung überlegen und haben eine verminderte antiandrogene und gestagene Nebenwirkung.

Die Antialdosteronwirkung wurde im Testmodell von Hollmann (Naunyn-Schmiedebergs Arch. Exp. Path. Pharmak. 247 (1964) Seite 419) gemessen.

In der folgenden Tabelle sind die relativen Werte der Antialdosteron-Wirkungsstärke(mit Kaliumcanrenoat = 1) der erfindungsgemäßen Verbindungen A, B und C zusammengestellt.

A 17β-Hydroxy-15β,16β-methylen-3-oxo-17α-pregna-1,4,6-trien-21-carbonsäure-Kaliumsalz,

B 1α,2α;15β,16β-Dimethylen-17β-hydroxy-3-oxo-17α-pregna-4,6-dien-21-carbonsäure-Kaliumsalz und

C 17β-Hydroxy-15α,16α-methylen-3-oxo-17α-pregna-4,6-dien-21-carbonsäure-Kaliumsalz.

**Tabelle**

| Verbindung | relative Antialdosteronwirkung |
|---|---|
| K-Canrenoat | 1 |
| A | 2 - 3 |
| B | 1 - 2 |
| C | 1 - 2 |

Die neuen Verbindungen sind zur Behandlung von Krankheitszuständen geeignet, an denen ein primärer oder sekundärer Hyperaldosteronismus beteiligt ist. Zu diesen Krankheitszuständen, die mit den neuen Verbindungen behandelt werden können, gehören zum Beispiel Lebercirrhose mit

Ascites und Ödemen, schwere Herzinsuffizenz, nephrotisches Syndrom, traumatisches Hirnödem usw.

Die Wirkstoffe werden vorzugsweise in wäßriger Lösung intravenös verabfolgt.

Die Dosierung der Wirkstoffe kann von Fall zu Fall variiert werden und hängt von Art und Schwere der Krankheit ab. Im allgemeinen wird die Wirkstoffmenge beim Menschen etwa 50 bis 600 mg pro Tag betragen.

Die wäßrige Lösung des Wirkstoffes kann in Ampullen, die 50 bis 200 mg Wirkstoff enthalten, aufbewahrt werden.

Die Herstellung der erfindungsgemäßen Verbindungen der allgemeinen Formel I erfolgt nach an sich bekannten Methoden aus den 21,17-Carbolactonen der allgemeinen Formel II durch Öffnung des Lactonringes.

Hierzu wird das Carbolacton mit einer äquimolaren Menge Alkalihydroxid in einem niederen Alkohol, wie zum Beispiel Methanol, Ethanol, Isopropanol, Propanol oder Butanol, auf etwa 50 bis 100°C erhitzt. Als Alkalihydroxide kommen Natrium-, Kalium- oder Lithiumhydroxid infrage.

Die als Ausgangsmaterial benutzten 21,17-Carbolactone der allgemeinen Formel II sind bekannt, zum Beispiel aus DE-OS 27 22 706.

**Beispiel 1:**

Zu einer Lösung von 3.0 g 15β,16β-Methylen-3-oxo-17α-pregna-1,4,6-trien-21,17-carbolacton in 45 ml 2-Propanol gibt man 9 ml einer 1N Kaliumhydroxidlösung in Methanol und kocht 30 Minuten am Rückfluß. Nach dem Abkühlen gibt man das Reaktionsgemisch in 45 ml eiskalten Diethylether, filtriert den Niederschlag ab und wäscht mit Diethylether nach. Der erhaltene Niederschlag wird anschließend mit Essigester ausgerührt, abgesaugt und im Vakuum getrocknet. Man erhält 2.6 g 17β-Hydroxy-15β,16β-methylen-3-oxo-17α-pregna-1,4,6-trien-21-carbonsäure-Kaliumsalz.

IR (KBr): 3410, 1650, 1600, 1580, 1400 $cm^{-1}$.

**Beispiel 2:**

Unter den im Beispiel 1 beschriebenen Bedingungen erhält man aus 300 mg 1α, 2α, 15β,16β-Dimethylen-3-oxo-17α-pregna-4,6-dien-21, 17-carbolacton 180 mg 1α,2α;15β, 16β-Dimethylen-17β-hydroxy-3-oxo-17α-pregna-4,6-dien-21-carbonsäure-Kaliumsalz vom Schmelzpunkt 183 - 186°C.

IR (KBr): 3420, 1645, 1575, 1400 $cm^{-1}$.

**Beispiel 3:**

Unter den im Beispiel 1 beschriebenen Bedingungen erhält man aus 1 g 15β,16β-Methylen-3-oxo-17α-pregna-4,6-dien-21,17-carbolacton 620 mg 17β-Hydroxy-15β, 16β-methylen-3-oxo-17α-pregna-4,6-dien-21-carbonsäure-Kaliumsalz.

IR (KBr): 3410, 1650, 1580, 1400 $cm^{-1}$.

**Beispiel 4:**

Unter den im Beispiel 1 beschriebenen Bedingungen erhält man aus 500 mg 15α, 16α-Methylen-3-oxo-17α-pregna-1,4,6-trien,21,17-carbo-lacton 335 mg 17β-Hydroxy-15α,16α-methylen-3-oxo-17α-pregna-1,4,6-trien-21-carbonsäure-Kaliumsalz.

UV: $\varepsilon_{283}$ = 22400.

**Beispiel 5:**

Unter den im Beispiel 1 beschriebenen Bedingungen erhält man aus 377 mg 1α,2α;15α,16α-Dimethylen-3-oxo-17α-pregna-4,6-dien-21,17-carbolacton 280 mg 1α,2α;15α,16α-Diemtyhlen-17β-hydroxy-3-oxo-17α-pregna-4,6-dien-21-carbonsäure-Kaliumsalz vom Schmelzpunkt 281 - 284°C.

UV: $\varepsilon_{283}$ = 17900.

**Beispiel 6:**

Unter den im Beispiel 1 beschriebenen Bedingungen erhält man aus 350 mg 15α,16α-Methylen-3-oxo-17α-pregna-4,6-dien-21,17-carbolacton 180 mg 17β-Hydroxy-15α,16α-methylen-3-oxo-17α-pregna-4,6-dien-21-carbonsäure-Kaliumsalz vom Schmelzpunkt 278 - 283° C.

UV: $\varepsilon_{283} = 24300$.

**Patentansprüche**

1. 15,16-Methylen-17α-pregna-4,6-dien-21-carbonsäuresalze der allgemeinen formel I

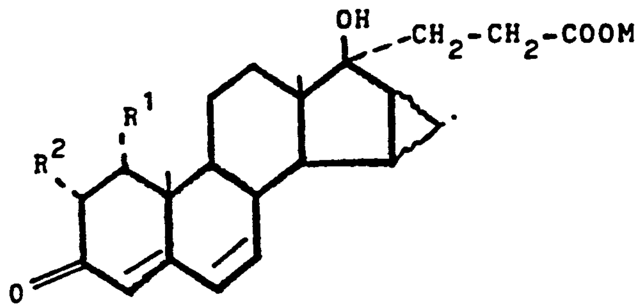

(I),

worin
R[1] und R[2] jedes für sich Wasserstoff oder gemeinsam eine weitere CC-Bindung oder eine Methylengruppe, und
M ein Alkalimetall
bedeuten
und die Methylengruppe in 15,16-Stellung in α- oder β-Stellung steht.

2. 17β-Hydroxy-15β,16β-methylen-3-oxo-17α-pregna-1,4,6-trien-21-carbonsäure-Kaliumsalz.
3. 1α,2α; 15β,16β-Dimethylen-17β-hydroxy-3-oxo-17α-pregna-4,6-dien-21-carbonsäure-Kaliumsalz.
4. 17β-Hydroxy-15β,16β-methylen-3-oxo-17α-pregna-4,6-dien-21-carbonsäure-Kaliumsalz.
5. 17β-Hydroxy-15α,16α-methylen-3-oxo-17α-pregna-1,4,6-trien-21-carbonsäure-Kaliumsalz.
6. 1α,3α;15α,16α-Dimethylen-17β-hydroxy-3-oxo-17α-pregna-4,6-dien-21-carbonsäure-Kaliumsalz.
7. 17β-Hydroxy-15α,16α-methylen-3-oxo-17α-pregna-4,6-dien-21-carbonsäure-Kaliumsalz.
8. Pharmazeutische Präparate, gekennzeichnet durch einen Gehalt an Verbindungen gemäß Anspruch 1 - 7.
9. Verfahren zur Herstellung von 15,16-Methylen-17α-pregna-4,6-dien-31-carbonsäuresalzen der allgemeinen Formel I, dadurch gekennzeichnet, daß man in an sich bekannter Weise auf ein 21,17-Carbolacton der allgemeinen formel II

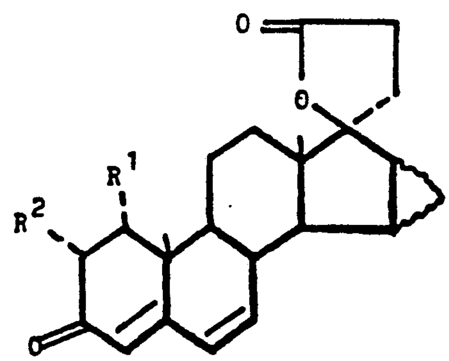

(II),

worin R[1] und R[2] die in Formel I angegebene Bedeutung heben, Alkalihydroxid einwirken läßt.

**Claims**

1. 15,16-Methylene-17α-pregna-4,6-diene-21-carboxylic acid salts of the general formula I

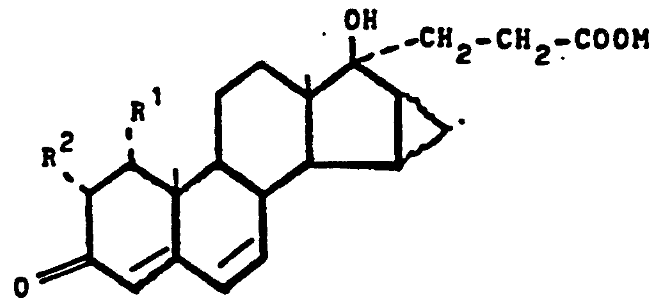

(I)

wherein

$R^1$ and $R^2$ are each hydrogen or together are another CC-bond or a methylene group,
and
M is an alkali metal
and the methylene group is in the α- or β-configuration.

2. Potassium 17β-hydroxy-15β,16β-methylene-3-oxo-17α-pregna-1,4,6-triene-21-carboxylate.
3. Potassium 1α,2α;15β,16β-dimethylene-17β-hydroxy-3-oxo-17α-pregna-4,6-diene-21-carboxylate.
4. Potassium 17β-hydroxy-15β,16β-methylene-3-oxo-17α-pregna-4,6-diene-21-carborylate.
5. Potassium 17β-hydroxy-15α,16α-methylene-3-oxo-17α-pregna-1,4,6-triene-21-carboxylate.
6. Potassium 1α,2α;15α,16α-dimethylene-17β-hydroxy-3-oxo-17α-pregna-4,6-diene-21-carboxylate.
7. Potassium 17β-hydroxy-15α,16α-methylene-3-oxo-17α-pregna-4,6-diene-21-carboxylate.
8. A pharmaceutical prepartion characterised by a content of a compound according to claims 1 to 7.
9. A process for the preparation of 15,16-methylene-17α-pregna-4,6-diene-21-carboxylic acid salts of the general formula I characterised in that, in known manner, a 21,17 carbolactone of the general formula II

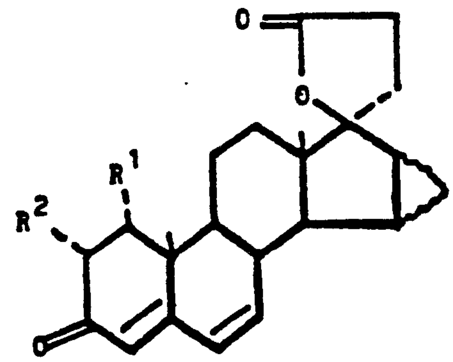

(II)

in which $R^1$ and $R^2$ have the meanings given in formula I, is reacted with alkali hydroxide.

**Revendications**

1. Sels d'acides méthylène-15,16 17α-prégna-diéne-4,6 carboxyliques-21 qui répondent à la formule générale I:

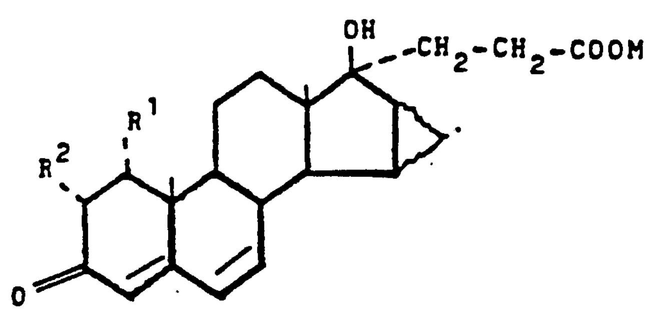

(I)

dans laquelle:

$R^1$ et $R^2$ représentent chacun un atome d'hydrogène ou forment ensemble une liaison carbone-carbone

supplémentaire ou un radical méthylène,
M représente un atome de métal alcalin et
le radical méthylène en 15,16 a la configuration α ou la configuration β.

2. Sel potassique de l'acide hydroxy-17β méthylène-15β,16β oxo-3 17α-prégnatriène-1,4,6 carboxylique-21.

3. Sel potassique de l'acide diméthylène-1α,2α;15β,16β hydroxy-17β oxo-3 17α-prégnadiène-4,6 carboxylique-21.

4. Sel potassique de l'acide hydroxy-17β méthylène-15β,16β oxo-3 17α-prégnadiène-4,6 carboxylique-21.

5. Sel potassique de l'acide hydroxy-17β méthylène-15α,16α oxo-3 17α-prégnatriène-1,4,6 carboxylique-21.

6. Sel potassique de l'acide diméthylène-1α,2α;15α,16α hydroxy-17-oxo-3 17α-prégnadiène-4,6 carboxylique-21.

7. Sel potassique de l'acide hydroxy-17β méthylène-15α,16α oxo-3 17α-prégnadiène-4,6 carboxylique-21.

8. Compositions pharmaceutiques caractérisées en ce qu'elles contiennent des composés selon l'une quelconque des revendications 1 à 7.

9. Procédé pour préparer des sels d'acides méthylène-15,16 17α-prégnadiène-4,6 carboxyliques-21 de formule générale I, procédé caractérisé en ce qu'on fait agir un hydroxyde de métal alcalin, de manière connue, sur une carbolactone-21,17 répondant à la formule générale II:

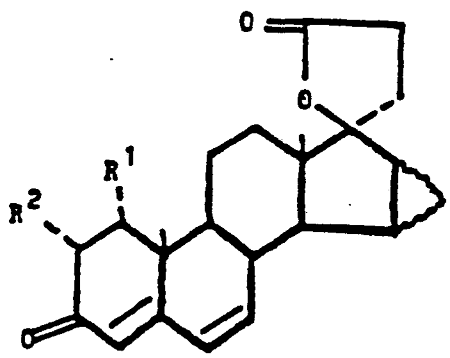

(II)

dans laquelle $R^1$ et $R^2$ ont les significations données à la revendication 1.